# EUROPEAN PATENT APPLICATION

(11) **EP 1 588 698 A2**
(43) Date of publication of application: **26.10.2005**
(21) Application number: 05251622.6
(22) Date of filing: 17.03.2005
(51) Int. Cl.: A61K 9/12

(54) **A process for the production and screening of materials for use in pharmaceutical aerosol formulations**

(30) Priority: 17.03.2004 GB 0406069
(71) Applicant: Thompson, James Anthony, Coleshill, Bucks HP7 0LG (GB)
(72) Inventor: Thompson, James Anthony, Coleshill, Bucks HP7 0LG (GB)

(57) **Abstract**

A process for the production and screening of materials for use in pharmaceutical aerosol formulations, and the subsequent manipulation of particle clouds and drug morphology.

## Description

### Scope of the invention

The present invention relates to pharmaceutical aerosol formulations, and in particular the processes of particle production, improved screening techniques for solution based aerosols, and the subsequent manipulation of aerosol cloud deposition patterns and morphology of drug derivatives.

### Background of the invention

Several methods have been utilised in generating aerosols in the particle size range requisite for respiratory therapy. Commercially, jet nebulisers, dry powder inhalers and propellant driven metered dose inhalers dominate the inhalation market. These systems are not, however, ideal delivery systems with each having inherent advantages and disadvantages. In achieving medicinal regulatory standards, they are usually simple in construction (with some exceptions), ergonomic, portable and relatively cheap to produce commercially. From a regulatory and, thus, a clinical viewpoint of acceptability, the aerosol generated must be below 10 microns and ideally within a well-defined size range depending on therapeutic application.

Selective or targeted deposition of inhaled pharmaceuticals to specific locations of the respiratory tract is preferred, as diseases are not distributed homogeneously throughout the lung. For example, cystic fibrosis may be distributed throughout the tracheobronchial tract. Asthma has been identified to localise within the large upper, central or smaller lower bronchial airways, whereas, at a specific regionalised locations carcinomas have a predilection for bifurcations. **Martonen, T., B. (1992). The behaviour of cigarette smoke in human airways.** ***Am Ind. Hyg*** **.Assoc.** ***J.*** **53: 6-15.**

More recently, the respiratory tract has been identified as route for systemic delivery, and, so, particle sizes of the therapeutic agents essential for enhanced delivery: for systemic delivery, particles should, ideally, be less than 1µm in MMAD. The peripheral regions of airways, the terminal bronchioles and the alveolar region, are separated from the systemic blood circulation via a very thin epithelium (<0.2µm). As deposition is dominated by diffusion in this region of the lung, the small particle size, coupled with large residence time and the large cross-sectional area of the alveoli make this an ideal delivery vehicle for portal entry.

The dose delivered from the system must be consistent and the respirable fraction suitable for the intended use. The packaging of the device and formulation is required to protect the physico-chemical integrity of the medicament and excipients, and not detrimentally interact with formulation, the system must also be easily transportable and be 'patient friendly'.

The current invention focuses on pressurised metered dose inhaler, but is in no way intended to be limited to this one inhalation dosage form, and those skilled in the art will appreciate technologies described can be equally applied to other forms via the inhaled route or equally other routes of administration: orally, bucally, topically parentally or the like. With respect to the pressurised metered dose inhaler flexibility can be designed into the invention from the disclosed information.

Currently, salbutamol as the sulphate salt derivative is generally formulated as a suspension in a pressurised metered dose inhaler, utilising a HFA propellant. Erstwhile CFC propellants are being phased out due to their detrimental effect on the environment: chlorofluorocarbons namely chloromethane and dichlorofluoroethane. Alternative hydrofluorocarbons HFA 134(a) (1, 1, 1, 2-Tetrafluoroetane) and hydrofluorocarbon 227ea (1, 1, 1, 2, 3, 3, 3-heptafluoroethane) are viewed as being less destructive to the ozone than their chlorofluorocarbon counterparts and, furthermore, they are toxilogically acceptable to mammalian based respiratory physiology. According to the prior art, therefore, salbutamol has been formulated as a suspension based product, in a two stage manufacturing process for use in aerosol preparations with non-chlorinated propellants. It is assumed persons of adequate knowledge and skill will have an appreciation of the dosage form, and will be aware of the intricacies of componentry and formulations. Several techniques are described in the literature to produce such systems **(See Purewal., T., S., and Grant., D.,** ***J*****., W. (1998). Metered dose inhaler technology. Interpharm Press Inc, Buffalo Grove, IL, USA).**

The majority of pressurized metered dose systems are formulated as a suspension in preference to a solution. Of the common marketed pMDIs only tornalate and QVAR to date are solutions **(Smyth, H., D., C. (2003). The influence of formulation variables on the performance of alternative propellant-driven metered dose inhalers.** ***Advanced Drug Delivery Reviews.*** **55: 807-828)**.These products are formulated with an ethanol co-solvent to aid dissolution of the active compound. Other solution-based systems are indicated in the literature in which the MMAD of the aerosol cloud can be directly modulated by the addition of non-volatile excipients: glycerol and PEG 400. Suspensions, which are unstable thermodynamic systems, are susceptible to several problematic areas. Depending on the formulation and metering valve, dose variations have been observed, **(Cyr, T., D., Duhaime, R., M., Graham**, **S., J., Ormsby E., D., Lawrence, R., C., and La Belle, M., J. (1997) Metered Dose Inhalers. Part 3. Metaproterenol Sulphate: Particle size distribution and dose uniformity. L.** ***Pharm. Biomed. Anal.*** **15:1709-1718),** coupled with canister orientation and environmental conditions crystal growth via Ostwald ripening can occur, which can lead to the dissolution of smaller suspended particles and the subsequent growth of larger crystals during storage. This may, concomitantly, lead to an increase in mean particle size of the delivered drug, and increase the potential for variations in deposition of the aerosol resulting in either drug over or under-dosing.

Other problems related with suspension based formulations, particularly with high drug loading, is the potential build up of drug on the actuator, which may block the spray orifice. As the drug in suspension systems are in a solid form, problems with polymorphism is a major concern. Polymorphism affects many intrinsic physico-chemical properties, and can increase solubility in the propellant/propellant systems enhancing the aforementioned problems.

Solution based pMDI systems would seem initially to be the preferred dosage form. However, there are difficulties formulating soluble ionic forms of drugs in organic solvent systems. Indeed it is has been found that the solubility of salt forms is generally lower in aprotic solvents than that of free bases or acids. This is affirmed from the level of ethanol in tornalate (Bitolterol Mesylate), presently at 38%w/w. Organic compounds, particularly steroids are suited to solution inhalers due to their solubility levels in both propellants and ethanol. This use of organic derivatisation to increase solubility is corroborated by prior studies: the increased solubility of a salbutamol ester in propellant 134(a).Seville, **P., C., Simons, G., Taylor, G., and Dickinson, P., A. (2000). Prodrug to probe solution HFA pMDI formulation and pulmonary esterase activity.** ***Int. J. Pharm.*** **195: 13-16.** Solution systems are obviously prone to chemical degradation as the drug is at the molecular level, but with appropriate use of theory and practice, soluble and stable salt solutions are possible. In addition to the in-situ, chemical, drawbacks experienced with solution-based systems data exists, which indicates that aerosol particles of this type of pressurised system is prone to greater changes in particle size than suspension-based counterparts. This is routinely considered to be a disadvantage with suspensions, as the particle size shifts, and exhibits a greater non-respirable fraction; but this has not been fully investigated in the solution setting. Changes in particle size ranges may, here, provide pharmaceutical benefits.

### Brief summary of the invention

The present invention provides advantages with respect to several areas of aerosol technology suitable for use within inhalation route. In one embodiment of the invention there is described a process for screening and production of particles either as a batch process at standard temperature or from a pressurised system i.e. at pressures greater than standard atmosphere. This screening technique effectively enables the feasibility of producing stable solution formulations or by manipulation of reaction thermodynamics and kinetics suspension or emulsion systems by precipitation of active(s) from solution(s) by use of an antisolvent or anti-solvents. The resulting use of screened derivatives can be then be utilised to change particle size distributions and morphology of the active to enhance and direct deposition.

Certain characteristics of the preferred formulations exhibit desirable physico-chemical stability, and can be manipulated for targeted delivery and may elicit extended duration depending upon the salt and the formulation.

The technique described may be utilised to produce particles for use in dry powder inhalers, nebulisers electrohdrodynamic systems or the like. For example, a number of actives and lactose excipients may be dissolved in a suitable solvent and crash precipitated from antisolvents such as ethers, esters, ketones, at room temperature and pressure or from pressurised anti-solvents such as Hydrofluoroalkanes Alkanes, Alkenes or compressed liquefied gases singly or in combination. In fact, any suitable compressed liquefied, or compressed gas in combination may be utilised. The temperature and pressure of reactants and or anti-solvents may be manipulated as required to alter particle size or morphology.

As a screening technique for solution-based inhalers toxilogically acceptable co-solvents agents such as ethanol or water or combinations can be utilised.

Solubility, of a drug salt derivative, to an appreciable extent, in solvents miscible with the propellant(s) provides a simple yet robust screening method. For example, taking the current example salbutamol sulphate manufactured by this method precipitated almost instantaneously from an ethanol solution. Several other salts also crystallised from solution rendering them suitable as suspensions. The maleate salt was stable as a solution in ethanol, even at ultra low temperatures and had to be precipitated by use of an antisolvent.

The use of novel derivates, salts in this case, can be utilised to manipulate two major characteristics of the aerosol cloud that has not been previously considered or indeed deemed possible by use of derivative only: the manipulation of particle size deposition patterns and the morphology of the medicament upon deposition i.e. its physical state whether amorphous solid or liquid "surfactant-like". Solution formulations are notorious as "finer" in terms of particle size judged by Mass median aerodynamic diameter (a log normal description aerosol particles) and not equivalent pharmaceutically to suspensions; this has lead to addition of large masses of non volatile excipients.

Another scope of the invention includes multiple combinations of active medicaments in solution, e.g. bronchodilator salt - steroid - parasymapthomimetic agent (salbutamol maleate-fluticasone propionate- ipratropium bromide) can be produced. High solid loadings of combination actives may lead to further manipulations of particle size distributions of the aerosol cloud.

Current medicaments may be modified, e.g. Salmeterol is currently delivered as the Xinafoate salt. The maleate or oleate my improve solubility to allow manipulation as a solution or precipitation as a suspension at a range of reaction temperatures or pressures.

Alternatively, some combinations may benefit from one or two drugs in solution and one or two in suspension. Skilled artisans will delineate such combinations. Probable combinations may incorporate the same drug as a mixture of solution and suspension so as to have a bi-modal distribution.

### Brief description of the drawings

Figure 1: A graphical representation of the theoretical and experimental solubility of salbutamol maleate and the theoretically predicted solubility of salbutamol oleate.
Figure 2: A graphical representation of the theoretical and experimental solubility of salbutamol stearate.
Figure 3: A Graphical illustration of the solubility of salbutamol maleate in pure 134(a).
Figure 4 : A Graphical illustration of the solubility of salbutamol oleate in pure 134(a).
Figure 5: A (side elevation) graphical illustration of the effect of solvent/antisolvent levels on drug stability and particle sizes of precipitated medicaments.
Figure 6: Plan view graphical illustration of the effect of solvent/antisolvent levels on drug stability.
Figure 7: Side elevation view of the stability of solutions systems after prolonged storage at ultra low temperature (-20°C for 6 months) and precipitation of drug in-situ utilising a combination of anti-solvents.
Figure 8: A graph illustrating the deposition profiles of salbutamol maleate and oleate at low temperature and high relative humidity.
Figure 9 : A graph illustrating the deposition profiles of salbutamol maleate and oleate at high temperature and low relative humidity.
Figure 10: A graph illustrating the deposition profiles of salbutamol maleate and oleate at low temperature and low relative humidity.
Figure 11: A graph illustrating the deposition profiles of salbutamol maleate and oleate at high temperature and high relative humidity.
Figure 12: A graph illustrating the deposition profiles of critical inhaler data: actuator deposition, throat deposition and fine particle fraction of a salbutamol maleate pressurized metered dose inhaler formulation.
Figure 13: A graph illustrating the deposition profiles of critical inhaler data: actuator deposition, throat deposition and fine particle fraction of a salbutamol oleate pressurized metered dose inhaler formulation.
Figure 14 : An SEM of salbutamol maleate particles deposited on a cascade impactor plate (stage 4).

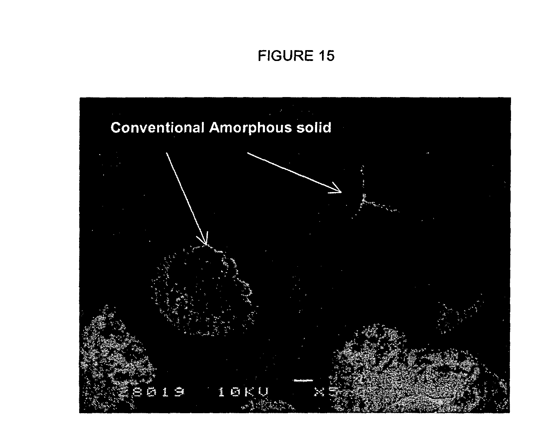

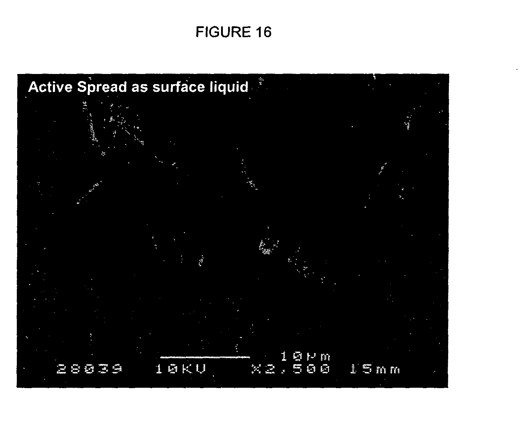

Figure 15: An SEM of salbutamol oleate particles deposited on a cascade impactor plate (stage 4).

### Detailed description of the invention.

It has been suggested from experimental investigations that the environment of the respiratory tract can influence the fate of inhaled aerosol particles. These influences have been observed from the deposition patterns that result from the physicochemical composition of the aerosol cloud, and the thermodynamic and kinetic interactions thereafter with the local environment: the temperature and humidity profiles of the respiratory tract. The human lung is essentially warm and humid, but it is however difficult to quantitate and map the localized temperature and humidity profiles throughout the system. In fact, it has been shown, the actual temperature and humidity profiles depend on the route of the passage of air: whether the air has been conditioned by nasal or oral breathing.

Preferred aerosol formulations of the invention comprise a therapeutic amount of a salt parent compound or derivative either singly or as a combination, a propellant or group of propellants selected from the group consisting of HFA 134(a) and or HFA 227 and mixtures thereof, and co-solvents, dispersants and excipients, in an amount to effect solubility of said salts and to dictate evaporation and hygroscopic processes. Other propellants may be taken from the following illustrative non-constrictive examples: decapentane, perfluorooctylbromide ... etc.

Changes in chemical composition of the formulation, both active and excipients, can then either accentuate or negate the effects of such interactions with water vapour molecules on resultant aerosol cloud emitted from the inhaler hardware. In the presence of varying degrees of water vapour many pharmaceutical preparations have been shown to be hygroscopic in nature, and strongly interact with water vapour present. Thus, upon the interaction of an aerosol particle with the water vapour, particles may change in size, shape and density. As a result, particle deposition both in terms of mass and specific location may markedly differ and clinical efficacy becomes effectively diminished.

The invention described discloses the steps that can be taken to manipulate and model the effects of particle deposition from first principles: from drug derivative synthesis, selection of stable pharmaceutical aerosol formulations, screened to produce solution or suspension derivatives either in bulk or in situ (the canister) and the subsequent effects on particle morphology that may allow manipulation of particle size depositions to deposit to selected locations of the lung. It has been further discovered that from such manipulations that the particle morphology can be altered to change physical state on deposition. Derivatives can be deposited as solids or possibly more advantageously as diffusely spread "liquid crystals", which is not apparent in the solid form. Surfactant like preparations, which phase transition at body temperature from conventional crystalline solids with elevated melting points/phase temperatures are not known, and is counterintuitive as phase transition temperatures of the solid usually dictate the physical form. A selection of derivatives are included in table 0.

To monitor the effects of hygroscopicity the first stage of experimentation dictated the synthesis of ethanol soluble derivatives of a model respiratory therapeutic. The beta₂ agonist salbutamol was chosen for this phase. This compound is preferred, but not intended to be in any way limiting as other active pharmaceutical ingredients may be employed. The derivatives chosen were selected as they covered extremes of water solubility.

### Derivative synthesis and formulation screening

The co-solvent used to screen derivative solubility and formulation approach was ethanol. Ethanol is a preferred co-solvent as it is toxilogically acceptable and miscible with HFA propellants, however, other acceptable co-solvents may be used for example glycerol, ployacohols, sugars isopropanol butanol etc. A comprehensive list can be taken from texts such as the handbook of pharmaceutical excipients. Ethanol and water mixtures may be utilised. For solution aerosols a single phase is required when in the final formulation, but equally a suspension or emulsion may results and be utilised. As the model bronchodilator was a basic compound acids requisite for salt formation (from pKa values) were selected. Organic acids proved suitable and are preferred but any suitable acid may be used. Further, it was discovered that carboxylic unsaturated organic acids were highly ethanol soluble although low molecular weight unsaturated acids such as butyric proved useful. During experimentation it was elucidated that in certain circumstances cis configured acid adducts indicated improved solubility over their trans counterparts: salbutamol maleate was ethanol soluble whereas salbutamol fumarate was not. A list of experimentally chosen acids are given in table 1 along with associated solubility expressed as a percentage weight per weight of solvent calculated from theory, (Clausius Clayperon relationship) but are in no way intended to be limiting. The solvent necessary to facilitate dissolution is assumed to be of sufficient dielectric constant and an ideal solvent, although skilled practitioners will endeavor to try a range of solvents as formulation science is empirical. Other examples of suitable acids are acetate, benzenesulphonate, mesylate etc, and can be sought from any suitable text: **Martindale 2005.** Indeed, several other salts have been manufactured some, of which, have been cited in he literature but never considered in pressurized metered dose inhalers. From the method of manufacture melting points as evaluated by DSC differed for some compounds (see table 0). This leads to an extended function of the invention: the use of polymorphs, clathrates solvates and also single enantiomers. Preferentially precipitated or solvated species may result from careful selection of reagents and forms of the active described. Preferentially precipitated or solvated compounds can be isolated either in bulk or in-situ for use.

For those well versed in the technology derivatives may be alternatively prepared by manipulation of the chemistry: esterification to produce suitable esters, production of ethers, polymerization reactions, preferential selection from keto-enol tautomerism and any suitable organic reaction mechanism.

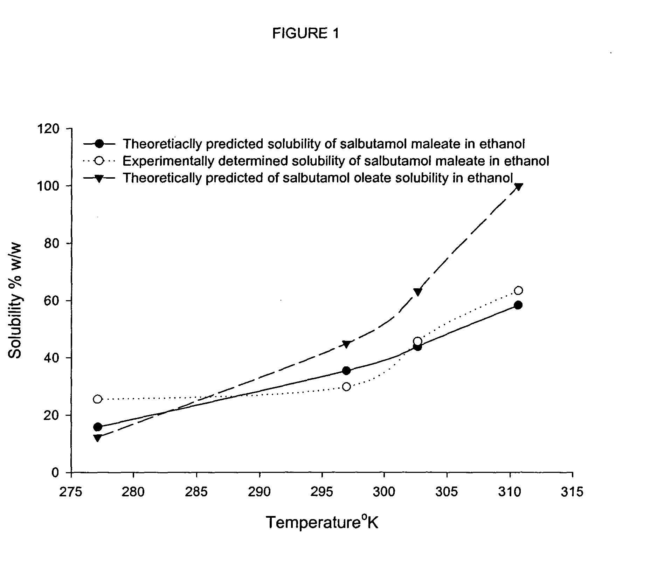

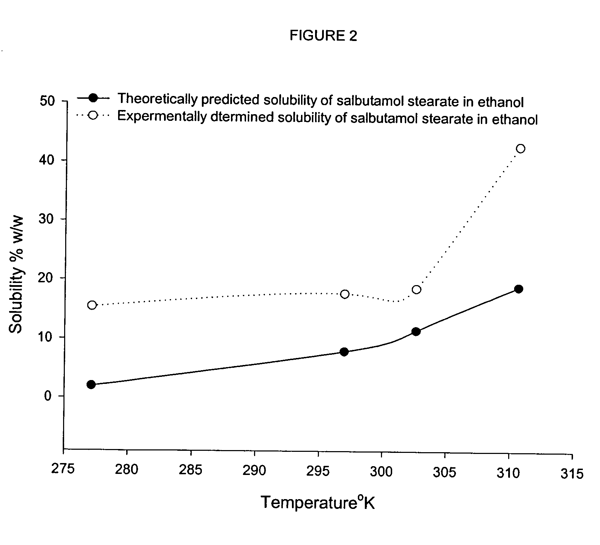

Theoretically calculated with practically measured solubilities are indicated in figures 1 and 2 for a selection of derivatives. Stable solution formulations are one embodiment of the invention since if a salt derivative does not precipitate from a reaction solution in which the crystallising solvent is ethanol it is inferred then a stable solution with pressurised liquefied gases is achievable. Two non-limiting examples of use of the manufacture/ screening method are described below.

### Example 1

### Synthesis of Salbutamol cis-butenedioate (Maleate) (BULK)

Approximately 0.5g (0.004 moles) of Maleic acid was dissolved in approximately 4ml (3.2g) of absolute ethanol and 3.2 g of diethyl ether by gentle warming and agitating for several minutes until complete dissolution had occurred. Approximately 1.0g (0.004 moles) of salbutamol base was subsequently added to the acid solution. The vessel was sealed and then warmed with agitation until the base had completely dissolved. The vessel was chilled for 24 hours at approx 2-8C to induce precipitation. The supernatant was removed and a further 5 ml of diethyl ether was added. The resultant oil was triturated until a white solid appeared. The solid was vacuum filtered and air-dried. By careful manipulation of the reagents the salt could be crystallised directly in the flask.

### Example 2 (IN-SITU)

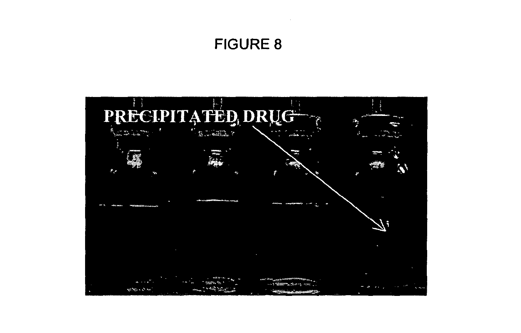

Alternatively, a solution of the salt can be prepared by stoichiometric reaction between drug and acid. Solution concentrates can be prepared over a range of concentrations for example from 0.1-100% w/w but can be adjusted for concentrations dependent on solubility, clinical efficacy, and temperature. These are then charged to the appropriate vessel in a required concentration and a metering valve crimped on the vessel. Depending on the concentration and volume of the solution antisolvent is charged through the vessel to initiate precipitation. The amount and rate can be manipulated to give desired character of particles ex-actuator. (See figure 8)

### Example 3

### Synthesis of Salbutamol cis-octadecenoate (Oleate)

Approximately 0.33g (0.001 moles) of oleic acid was dissolved in 10ml of acetone with warming. To this solution was added approximately 0.25g (0.001 moles) of salbutamol base, which formed a milky suspension formed. The vessel was sealed and the suspension heated with serial venting until complete dissolution of the base. The solution was then chilled, and an oil then formed, which was re-dissolved by agitation at room temperature. A white precipitate formed after several minutes.

The three examples above are illustrative, and it is recognised that adept practitioners in the art will try a plethora of solvent combinations and additives to enhance either solubility or induce crystallisation. As supplementary personifications, drug salt adducts may be two active compounds ultimately forming a single drug. By way of a non-restrictive example a basic compound such as salbutamol and an acidic compound such as cromoglycic acid would potentially form salbutamol cromoglycate.

Alternatively, bronchodilators and steroids may be employed. Other auxiliary examples may comprise one or more active compounds in solution but co medicaments may be in solution or suspension. Such further actives may include but not be limited to analgesics such as codeine, morphine Tetrahydocannabinol or dihydrocodeine; anti allergics such as cromoglycate or nedocromil; antibiotics such as penecillins, cephamycin, sulphonamides or tetracyclines; antihistamines such as methapyrilines; antitussives such as noscapine, xanthines or thephylines; steroids such as beclomethasone diproiponate, fluticasone, dexamethasone and the like; parasympathomimetics such as ipratropium bromide and oxitrpium bromide and tiotropium bromide; therapeutic peptides and proteins or amino acids such as insulin and anticancer agents such as methotreaxate and tamoxifen.

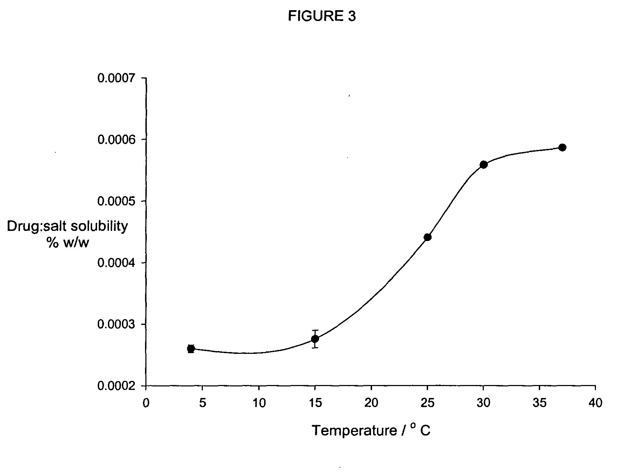

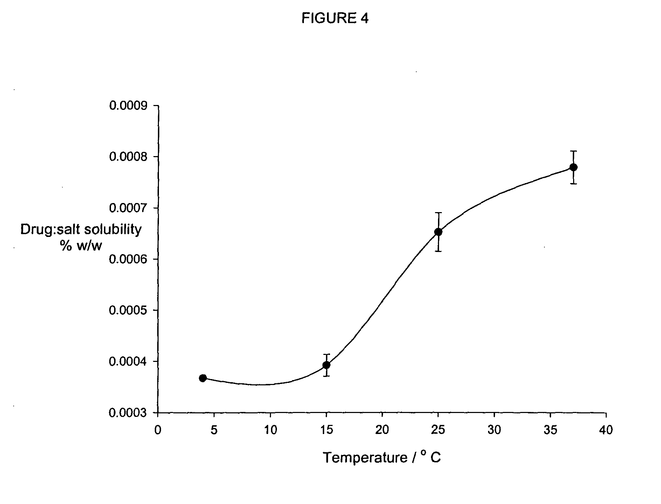

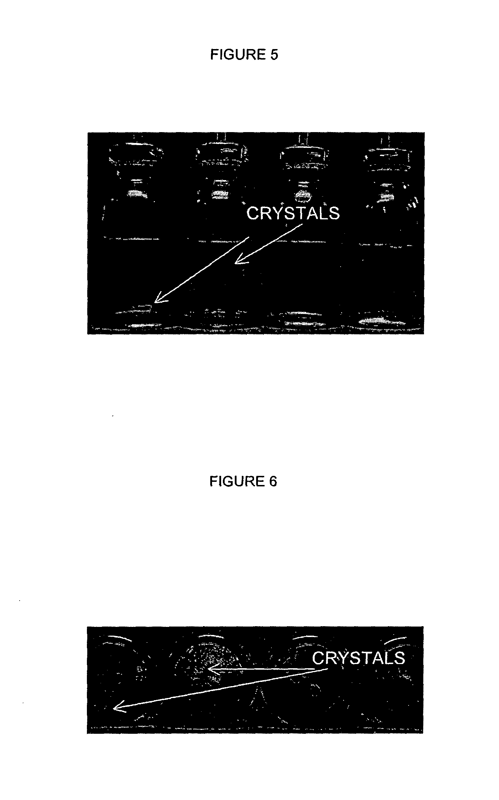

The solid compound is then isolated by a suitable method. Figures 3 and 4 indicates the solubility profiles of the respective salts synthesised. To formulate stable ethanol soluble derivatives an empirical approach was taken whereby formulations were prepared in plastic coated glass bottles with co-solvent levels ranging from 1-50% w/w respectively. The mass of derivative was formulated at a level to give an equivalent to 100mcg of free base per actuation from the metering valve. Results from the formulations are depicted in figure 5 for the maleate salt 6 for the oleate salt and 7 to illustrate the precipitation technique by 7 controlled precipitation by the use of a second antisolvent: water on formulations of salbutamol maleate.

Evaluation of the results displayed stable solution formulations of both salts achievable at 15% w/w co-solvent level for the maleate and 30%w/w for the oleate. Skilled artisans may endeavour to include surfactants or further co-solvents to enhance solubility. Such excipients may include but not be limited to conventional surfactants: Brij's Tween's lecithins polyvinylpyrollidone and those described in suitable pharmaceutical excipients handbooks. Figures 5, 6 (maleate salt side and plan views) and 7 (oleate salt arrows indicate crystals) illustrate 134(a) as an antisolvent as the drug crystallises from solution. The particle size of each drug is different, which leads to an embodiment of invention: use of derivative, co-solvent level, excipients level and antisolvent concentrations to precipitate actives to a desired particle size by careful moderation of the components.

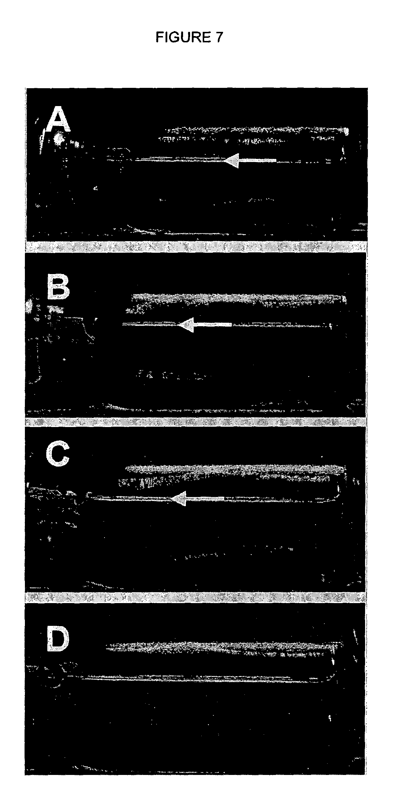

In figure 7 the use of a further embodiment of the invention is described: a solution aerosol is formed and a controlled precipitation is produced from a combination of anti-solvents. In this embodiment the drug precipitates from a HFA 134(a) and water mixture. This is a novel, as these two agents (water and 134a) are routinely kept apart for a variety of formulation issues. The solution was stable at room temperature but precipitated at low temperatures particularly < -20°C. This leads to facilitate those skilled in the art to try various combinations and permutations of reaction conditions. The nature/chemical composition of the reagents, temperature of reagents, pressure of reagents and order of addition can all be utilised to give the desired particle size. The use of water is a further embodiment of the invention. In small concentrations water is soluble in the new HFA propellants. As an example actives with a high solubility may be dissolved in water and "crash precipitated" by use of a suitable anti-solvents erstwhile illustrated.

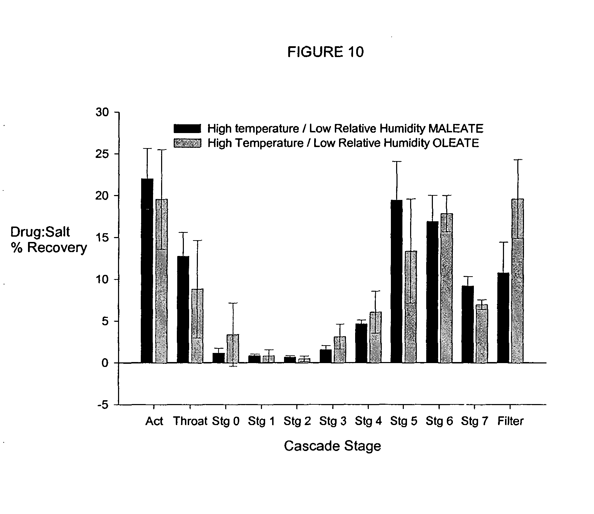

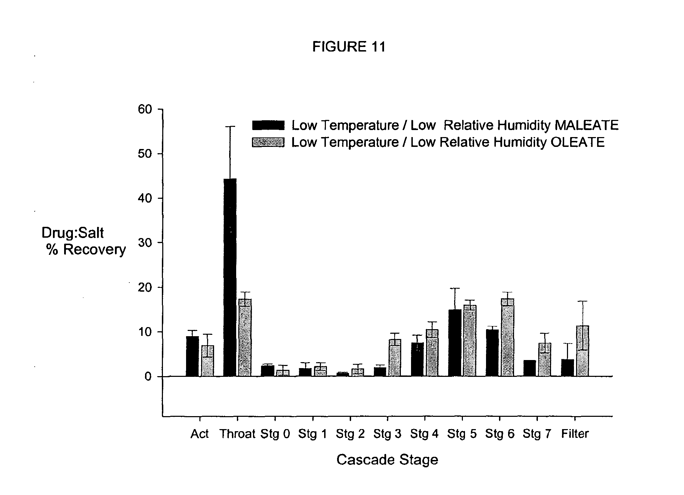

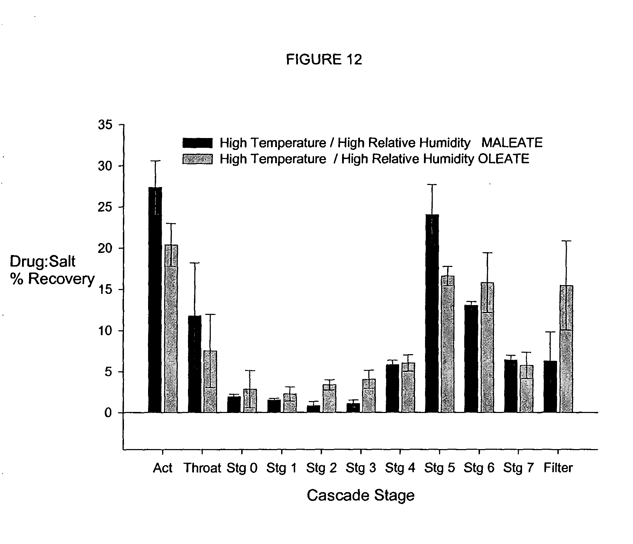

For example, figures 8-13 delineate a range of particle sizes resulting from a selection of drug and said reaction conditions after emanation from a metered dose inhaler. Precipitated drug and excipients combinations can be left as an in situ formulation or the anti-solvents removed and the powdered medicament used externally: within a dry powder inhaler. Drug excipients combinations manufactured from this method may allow improvements in powder flow and de-aggregation and improved stability of medicaments that are affected by moisture. Indeed it may be that those skilled in the art may produce particle for use in other dosage forms not related to inhalation therapy as the method of manufacture is suitable for thermally labile compounds.

The following are non restrictive examples of formulations using either solution or suspension technologies:

### Example 4

| **Solution formulation: At RT (approximate values)** | | |
|---|---|---|
| Component | Mass / g | % w/w |
| Salbutamol Maleate | 0.025 | 0.242 |
| Ethanol | 1.50 | 14.5 |
| 134(a) | 8.50 | 82.32 |
| Water | 0.3 | 2.91 |

The above formulation forms a suspension when stored at sub-zero temperatures indicating that temperature of components are important during particle size of formulations

### Example 5

| **Solution formulation: At RT (approximate values)** | | |
|---|---|---|
| Component | Mass / g | % w/w |
| Salbutamol Maleate | 0.025 | 0.242 |
| Ethanol | 1.50 | 14.5 |
| 134(a) | 8.50 | 82.3 |

### Example 6

| **Suspension formulation: At RT (approximate values)** | | |
|---|---|---|
| Component | Mass / g | % w/w |
| Salbutamol Maleate | 0.016 | 0.16 |
| Ethanol | 0.053 | 0.53 |
| 134(a) | 9.931 | 99.31 |

### Aerosol cloud particle size and morphology manipulation

It is well-known that formulation parameters including the delivery hardware influence resulting particle size of a propellant driven. By doing so, the resulting MMAD for a suspension-based inhaler could be manipulated. The aforementioned criteria have some credence when solution systems are considered, but several deviations occur. The main problems with solution systems are the 'finer' particle size and their resulting implications related to therapeutic index **(June, D. (1997) Achieving the change: Challenges and successes in the formulation of CFC-free pMDI's.** ***Eur Respir. Rev.*** **7: 32-34.).** In conjunction, devices are currently tested under laboratory environments by a number of sizing methods, which measure the aerosol particle size fractions *in-situ* or by fractionating into particle size ranges. This allows the calculation of the average particle size, the total drug recovered, and the residual drug deposited on the device/actuator. There are, however, two major problems with the testing of aerosols by current pharmacoepial methods: firstly, when aerosols are measured by different methods they often yield different information and, secondly, all measurements are made at ambient conditions of temperature and relative humidity. As the human body and respiratory tract essentially operates at 37°C and 99.5 % relative humidity, although temperature and humidity gradients are know to exist, then characteristic measurements of aerosol delivery should be made under or approaching these conditions.

It would be advantageous therefore to be able to manipulate aerosol cloud dynamics with" minimum formulation" requirements: without the use of additional non-volatile excipients such as glycerol although those in the art may wish to incorporate them into formulations. Disclosed and hitherto unknown are two derivatives formulated which produce aerosol particle size distributions contrary to what is expected. Further when tested under varying conditions of temperature and humidity the resultant clouds change in character. To complement this, it has been also discovered that certain specifications of the aerosol deposition change, and it would be advantageous to incorporate varying water a contents and temperatures of make up air to the aerosol.

### Non-limiting example of formulation preparation

Formulations of salt derivatives whether prepared by the following method but any suitable method can be utilise. Plastic coated glass pMDI bottles (St Gobain, France) were weighed on a balance and tared after first undergoing a rinse with clean and dry compressed air. Drug was weighed accurately by difference in order to give an equivalent of 100mcg freebase as related to molecular mass of the salt and the same procedure repeated for absolute ethanol (Fisher scientific). A continuous sprat valve was crimped on (Bespak, Kings Lynn, UK), according to manufacturers specifications and the bottle then sonicated (Decon FS 3006) for several minutes to induce complete dissolution of the drug. The bottle was then dried and once again tared on the balance. HFA 134(a) was subsequently charged through the valve using a Pamasol 2002 hand gasser crimper (DH Industries, Laindon, Essex, UK). The final formulation preparation was sonicated for a further several minutes for completeness. Formulations were then stored at specified temperatures and assessed periodically. All solutions were prepared in duplicate. 10% w/w ethanol : 90% w/w HFA 134(a) formulations were prepared. This is in no way intended to limit the extent of the formulations.

### Manipulation of critical particle size

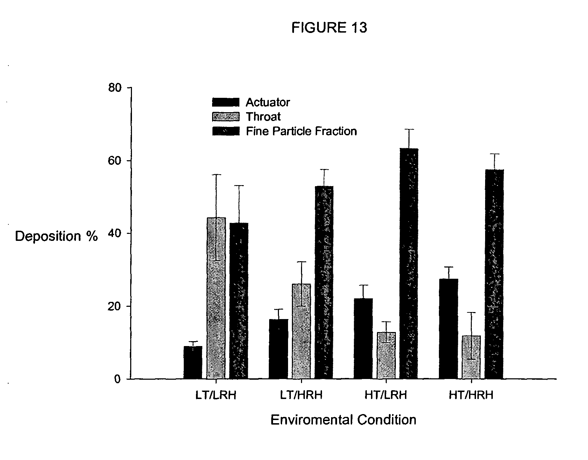

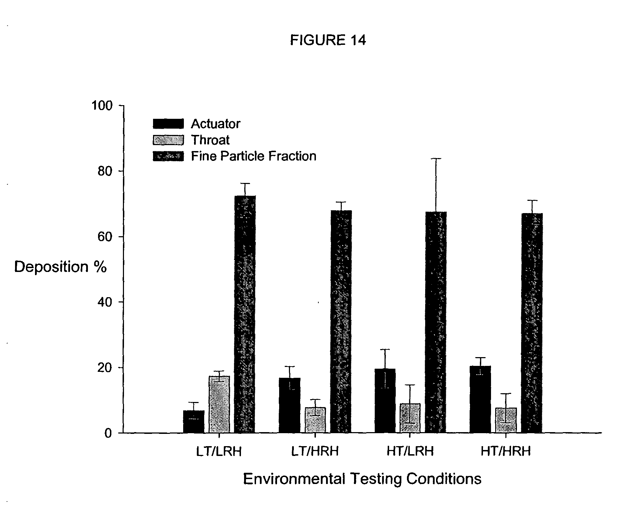

Tables 2,3 and 4 and figures 13 and 14 indicate the effects of salt selection on critical particle size parameters. Throat deposition and fine particle fraction are two major descriptors of emitted aerosol clouds. For a water insoluble derivative the fine particle fraction is unaffected by environment, whereas the water-soluble derivative was. It is therefore a preferred incarnation of the invention to select a derivative that if desired might negate the undesirable effects of hygroscopic growth. Alternatively a reduced deposition may be required, which can be achieved by utilisation of said derivatives. In a preferred embodiment reduced throat deposition of drugs is possible; for drugs that cause candiasis e.g. steroids this would be of immense benefit. From the data generated metered dose inhalers of this type would benefit from high temperature and humidity of intake air along with the aerosol.

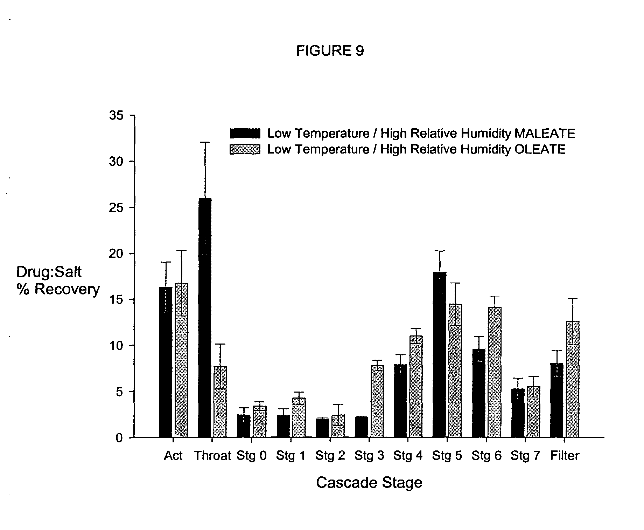

Solutions were tested for particle size using a cascade impactor, which to that practitioner skilled is standardised method for determining particle size distributions, under conditions of thermal and environmental control. Figures 9-12 illustrate the invention from particle size results. Both salt forms should when formulated at a low co-solvent should have resulted in deposition patterns similar to the oleate: small MMAD and patterns centred on stages 6,7, and filter of the impactor. Surprisingly, both salts gave different deposition patterns indicated when tested under differing conditions illustrated in table 2. It is therefore an embodiment to use derivatives to utilise derivatives not known to optimise deposition.

It was also noted that both temperature and co-solvent level affected the MMAD and changing the co-solvent level and temperature further affected the MMAD:

At 15% w/w co-solvent and 4°C the MMAD was 2.08 microns and 37° C the MMAD was 1.67 microns, whereas at 20% w/w co-solvent and 4°C the MMAD was 2.30 microns and 37° C the MMAD was 1.96 microns respectively.

### Manipulation of particle morphology

Particles that emanate from varying forms of inhalers exhibit differing morphologies. For example, particles emitted from dry powder inhalers consist of crystals of peculiar habits related to the active and lactose carriers. Depending on the drug and blend of carrier the particles these will exhibit changes in morphology and associated deposition patterns when they interact with warm and humid conditions of the human lung. Those from pressurised metered dose inhalers are effectively equivalent to spray dried particles and as such are amorphous solids. As such these deposit at specific locations of the lung as indicated from in vitro measurements via andersen cascade impactor. As an extended example of the invention it was discovered that drug salt derivative changed the morphology of the particles. Those synthesised from the fatty acid oleic acid, which has been a conventional surfactant for CFC inhalers and used as a valve lubricant indicated a liquid and not a solid particle and had been modified and had acquired surfactant like nature. Surfactants have been routinely used in respiratory medicine. **Morley. C. J., Bangham. A.B., Miller. N., Davis. J.A. (1981) Dry artificial lung surfactant and its effect on very premature babies.** ***The Lancet.*** As such compounds such as phospholipids are essentially lyotropic liquid crystals and as such are influenced by solvent interactions; depending on the concentration of lipid, solvent-induced aggregation of the constituents results in a complex range of structures. Formulations of which have been manufactured to phase transition and spread at an air water interface. Unexpectedly, the oleate salt spread as a surfactant, but had a higher phase transition temperature than the lab ambient and did so without a liquid air interface. This spreading ability it is theorised can be utilised to deliver active medicaments to cover more receptors and negotiate obstructions in the lung to reach its desired target

**Table 0**

| **SALT ANION** | **MELTING POINT (approx.) °C** |
|---|---|
| Acetate | ND |
| Fumarate | ND |
| Lactate | ND |
| | |
| NB the Fumarate was insoluble in ethanol even though its only difference to the maleate was the trans arrangement of the carboxylic acid groups. | |
| | |
| Adipate | 192 |
| Stearate | 108 |
| | |
| NB The adipate and state mp differs from that described by Jashnani et al probably due to the solvents used in the synthesis | |
| | |
| Mesylate | Degrades after 124 |
| Besylate | Degrades after 138 |
| Tosylate | Approx 160 |
| Succinate | 186 |
| Nitrate | 147 |
| Phosphate | 201 |
| Hydrochloride | 188 |
| Maleate | 130 |
| Sulphate | 200 |
| Oleate | 77 |

**Table 1:**

| Salt Derivative | Theoretical Solubility % w/w | | | |
|---|---|---|---|---|
| | 0°C | 4°C | 25°C | 37°C |
| Maleate | 13.231 | 15.797 | 37.047 | 57.251 |
| Stearate | 1.417 | 1.931 | 8.559 | 18.307 |
| Oleate | 9.261 | 12.305 | 48.280 | 97.033 |

**Table 2:**

| Drug derivative | Low Temp Low RH | Low Temp High RH | High Temp Low RH | High Temp High RH |
|---|---|---|---|---|
| Maleate | 1.48 | 1.35 | 1.11 | 1.21 |
| Oleate | 1.27 | 1.42 | 0.87 | 1.13 |

**Table 3**

| %Deposited at Location | LT/LRH | LT/HRH | HT/LRH | HT/HRH |
|---|---|---|---|---|
| ACTUATOR | 8.97 | 16.33 | 22.00 | 27.35 |
| THROAT | 44.24 | 25.96 | 12.74 | 11.78 |
| FPF | 42.76 | 52.88 | 63.24 | 57.42 |

**Table 4**

| % Deposited at Location | LT/LRH | HT/LRH | LT/HRH | HT/HRH |
|---|---|---|---|---|
| ACTUATOR | 6.86 | 16.74 | 19.54 | 20.37 |
| THROAT | 17.29 | 7.70 | 8.82 | 7.52 |
| FPF | 72.33 | 67.87 | 67.43 | 67.00 |

## Claims

1. A process for the production and screening of materials for use in pharmaceutical formulations and the subsequent manipulation of materials either as raw component or final pharmaceutical dosage form comprising of the following steps:
(a) Formation of a solution of a pharmaceutical acceptable substance or mixture of substances in a suitable solvent or mixture of solvents and excipients,
(b) Formation of a derivative or derivatives that have enhanced solubility in said solvent(s), from a selection of suitable chemical reaction or reactions,
(c) Manipulation of the reaction conditions (temperature and pressure) to enhance production of said derivatives,
(d) Manipulation of reactants to enable selection of solution formulations,
(e) Manipulation of reactants and anti-solvent(s) to precipitate said derivative(s) that can be subsequently utilised as a formulation in its own right, e.g. in-situ precipitation into an aerosol container
(f) Removal of reactant and isolation of derivatives(s) from (e) to produce particles for use in alternative dosage form or to be returned into the initial dosage form at a later date,
(g) Manipulation of the morphology and particle size of the acceptable substance(s) delivered from the dosage form.

2. The process of claim 1 wherein the solution contains an active pharmaceutical(s), which has an improved solubility in the solvent(s) by derivatisation particularly from a salt adduct reaction.

3. The process of claim 2 wherein the active pharmaceutical(s) can be precipitated from the solvent(s) by conventional precipitation at room temperature and pressure.

4. The process of claim 3 wherein the solution contains added excipients and precipitation can be appropriated from solution via a pressurised antisolvent.

5. A process in claims 1-4 where the dosage form is a pressurised metered dose inhaler

6. A process in claims 1-2 where the active is sufficiently solubilised within the system and the dosage form is a solution based metered dose inhaler,

7. A process in claim 6 where the solubilised active can be precipitated by addition of an antisolvent under pressure and thus the dosage form is a suspension based metered dose inhaler.

8. A process in 1-4 wherein the active and excipients can be isolated and utilised in another dosage form: pulmonary (e.g.dry powder inhaler, nebuliser) nasal delivery, buccal delivery, topical delivery, orally and parentally.

9. The process in claim 1 where one or more medicaments is salbutamol

10. The process in claim 1 where one or more solvents / dispersants are ethanol

11. The process in claim 10 where one or more solvents / dispersants are water

12. The process in claims 1-4 where one or more solvents are 134(a) or 227ea

13. A salt adduct formed from an organic acid, as per claims 1-4

14. A salt adduct formed from a carboxylic acid, as per claims 1-4

15. A salt adduct formed from an unsaturated cis configured carboxylic acid, as per claims 1-4.

16. A derivative formed from an ester, solvate, clathrates or polymorph, as per claims 1-4.

17. A derivative of a single enantiomer, as per claims 1-4

18. A combination formulation of drugs of varying receptor category e.g. bronchodilator and steroid.

19. A derivative formed from the reaction between two suitable actives forming an adduct e.g. salbutamol and cromoglycic acid forming salbutamol cromoglycate.

20. A process from claims 1-8 where the size is acceptable for pulmonary delivery: 0.5-5 microns

21. A process from claims 1-8 where the size is acceptable for nasal delivery: 1-500microns.

22. A process whereby the particle size distribution of a pressurised metered dose aerosol formulation is of a distribution similar to a suspension formulation without excessive non-volatile excipients.

23. A process whereby the use of increased ethanol/water co-solvents in a solution formulation modifies the particle size distribution further to that in claim 22.

24. The use of a salt medicament to alter the particle size of a pressurised metered dose aerosol formulation solution.

25. The use of a salt derivative formed from a surface-active agent enabling a change in particle morphology post aerosolisation to spread over a tissue surface.

26. The use of temperature and humidity of intake air to reduce actuator and throat deposition from metered dose inhalers.

27. The use of a derivative that negates the adverse effects of temperature and humidity of physiologic conditions maintaining constant fine particle dose deposition from metered dose inhalers.

28. The claim as in 27 where no excipients are utilised to negate the effects of temperature and humidity purely derivative alone.
